# EUROPÄISCHE PATENTSCHRIFT

(11) **EP 0 864 861 B1**
(45) Veröffentlichungstag und Bekanntmachung des Hinweises auf die Patenterteilung: **10.11.2004**
(21) Anmeldenummer: 98103004.2
(22) Anmeldetag: 20.02.1998
(51) Int. Cl.: G01N 33/00

(54) **Messanordnung zur Untersuchung gasförmiger Medien**
Measurement arrangement for examining gaseous media
Dispositif de mesure pour examiner des milieux gazeux

(30) Priorität: 14.03.1997 DE 19710527
(43) Veröffentlichungstag der Anmeldung: 16.09.1998
(73) Patentinhaber: Testo AG, 75853 Lenzkirch (DE)
(72) Erfinder: Demisch, Ulrich, Dr. Dipl.-Phys., 79102 Freiburg (DE); Ziegler, Peter, Dipl.-Phys., 79853 Lenzkirch (DE); Finbow, John, East Wellow, Hants 5051, 6 (GB); Sullivan, Peter, Chichester, West Sussex, P020 6 (GB)
(74) Vertreter: Patentanwälte Westphal, Mussgnug & Partner

(56) Entgegenhaltungen:
- EP-A- 0 132 970
- EP-A- 0 522 845
- DE-A- 4 417 665
- US-A- 4 689 136
- US-A- 4 827 778
- PATENT ABSTRACTS OF JAPAN vol. 014, no. 309 (P-1071), 4.Juli 1990 & JP 02 098660 A (FUJIKURA LTD), 11.April 1990,

## Beschreibung

Die Erfindung betrifft eine Meßanordnung zur Untersuchung gasförmiger Medien gemäß dem Oberbegriff des Anspruchs 1.

Elektrochemische Gassensoren, die zum Nachweis und zum Messen der Konzentration von Sauerstoff oder toxischen Gasen, wie z. B. CO, NO, NO2, SO2, geignet sind, sind als im Handel erhältliche Bauteile seit langem bekannt und beispielsweise in der DE 44 17 665 A1 als Bestandteil einer Meßanordnung beschrieben, von der die Erfindung ausgeht.

Die Meßanordnung besteht aus einem Sensorelement, welches elektrische Signale entsprechend der Konzentration und/oder der Zusammensetzung des Gases erzeugt. Das Gas wird dem Sensorelement über ein Gaszuführungselement zugeleitet und von diesem über ein Gasabführungselement abgeleitet.

In Bezug auf die Art und Weise, wie das nachzuweisende Gas an und in den Sensor gelangt, sind zwei Transportmechanismen zu unterscheiden. Einerseits wird das zu analysierende Gas oder Gasgemisch mittels einer Pumpe an den Sensor herangeführt. Andererseits gelangt ein Teil dieses herangeführten Gases über einen Diffusionsvorgang in das Innere des Sensorelements, und dient der Signalerzeugung.

Die Diffusion erfolgt aufgrund eines Gas-Konzentrationsgefälles zu einer Arbeitselektrode hin, die sich im Inneren des Sensors befindet. Die Arbeitselektrode setzt das Gas in einer chemischen Reaktion um und wirkt wie eine Senke.

Bei einer Reihe von Anwendungsfällen, wie beispielsweise bei der Analyse von Rauchgas von Verbrennungs- oder Heizungsanlagen, kann der Sensor aufgrund der herrschenden Umgebungsbedingungen, wie beispielsweise hoher Temperatur oder agressiver Begleitstoffe, nicht direkt am Meßort eingesetzt werden. In derartigen Fällen muß das zu analysierende Gas mittels einer Transporteinrichtung, beispielsweise der eingangs erwähnten Pumpe, zum Sensor gefördert werden, bevor es dem Sensorelement über die beschriebenen Diffusionsmechanismen zugeführt werden kann.

Problematisch ist in diesem Zusammenhang, daß zum Transport des zu analysierenden Gases Pumpen, wie beispielsweise Membranpumpen, verwendet werden, die Druckstöße im geförderten Gasstrom erzeugen. Durch diese Druckstöße wird der Diffusionsmechanismus empfindlich gestört, da die von den Druckstößen herrührenden Konzentrations- oder Dichteschwankungen zu konvektiven Massenströmen des Gases führen, die sich mit dem Diffusionsprozeß überlagern. Als Folge davon wird das Meßergebnis infolge eines stark überhöhten oder unkontrolliert schwankenden Sensorsignales verfälscht. Je nach Aufbau des Sensorelements ist eine mehr oder weniger stark ausgeprägte Druckstoßempfindlichkeit festzustellen.

Zur Lösung dieses Problems erscheint es deshalb zweckmäßig, Pumpen zum Transport des Gases einzusetzen, die einen möglichst gleichförmigen Gasstrom ohne Druckstöße oder Pulsationen liefern. Solche Pumpen sind jedoch aus ökonomischen Gründen für viele Anwendungsfälle nicht einsetzbar. Auch besitzen sie in der Regel ein großes Bauvolumen, so daß insbesondere bei transportablen Gasanalyseeinrichtungen solche Lösungen nicht realisierbar sind.

Um weiterhin die äußerst kostengünstigen und kleinbauenden Membranpumpen verwenden zu können, müssen spezielle Maßnahmen vorgesehen werden, um die für diese Pumpen typischen Druckstöße zu minimieren. In der Praxis werden deshalb Dämpfungselemente vorgesehen, die im Gasweg zwischen der Pumpe und dem Sensor eingebaut sind. In der Firmenschrift der Anmelderin "Rauchgasanalyse für Praktiker" ist ein solches Dämpfungselement in Form einer Kapillarenkammer dargestellt. Die Kapillaren wirken hierbei als Strömungswiderstand und bedämpfen die Druckstöße weitgehend. Hinter der Kapillarenkammer ist eine weitere Kammer vorgesehen, die aufgrund ihres Volumens als Pufferkammer wirkt und zu einer weiteren Vergleichmäßigung des Gasstromes beiträgt.

Diese Art der Dämpfung von Druckstößen ist zwar bestens geeignet, eine Signalverfälschung durch Druckstöße weitgehend auszuschalten. Die Anwendung dieser Anordnung ist jedoch insbesondere für stationäre Anlagen vorbehalten, bei denen das große Bauvolumen und das hohe Gewicht keine entscheidende Rolle spielt. Für den mobilen Einsatz, insbesondere für kleine handgehaltene Gasanalysegeräte, wie sie beispielsweise von Schornsteinfegern zur Überprüfung von Heizungsanlagen verwendet werden, ist dieses Konzept nur unter erhöhtem Kostenaufwand und Patzbedarf realisierbar.

Es wurde deshalb bereits der Versuch unternommen, eine Dämpfungswirkung durch Verlängerung des Gaswegs zwischen der Pumpe und dem Sensorelement zu erzielen. Die Druckstöße sollten hierbei aufgrund innerer Reibung soweit abgebaut werden, dass keine unzulässigen Signalverfälschungen mehr auftreten. Es hat sich allerdings gezeigt, dass neben den damit verbundenen Platz- und Kostennachteilen eine überproportionale Verlängerung der Systemansprechzeit entsteht, die in der Praxis nicht hinnehmbar ist.

Aus der EP 132970, von der die Erfindung ausgeht, ist es bekannt, das Dämpfungselement in Form einer Expansionskammer in das Sensorgehäuse zu integrieren und damit einen extrem kompakten Aufbau zu realisieren. Das Gehäuse nimmt neben dem eigentlichen Sensorelement darüber hinaus das Gaszuführungselement und das Gasabführungselement auf. Das Dämpfungselement ist somit integraler Bestandteil dieser Baugruppe.

Der Erfindung lag daher das Problem zugrunde, eine Messanordnung der eingangs beschriebenen Art derart weiterzuentwickeln, dass sie die geschilderten Nachteile nicht mehr aufweist.

Gelöst wird dieses Problem mit einer Messanordnung, die die Merkmale des Anspruchs 1 aufweist. Vorteilhafte Varianten dieser Messanordnung sind durch die Merkmale der Unteransprüche definiert.

Die Erfindung sieht vor, die Expansionskammer in einen Gehäusedeckel einzuformen, der auf ein das Sensorelement aufnehmendes Gehäuseunterteil aufgesetzt ist. Weiterhin trägt der Gehäusedeckel das Gaszuführungselement und das Gasabführungselement, die beispielsweise als Schlauchnippel ausgebildet sind. Der Gehäusedeckel lässt sich als Spritzgießteil kostengünstig herstellen und erlaubt darüber hinaus die leichte Zugänglichkeit zu dem Sensorelement.

Eine besonders geringe Signalverfälschung ist dann zu beobachten, wenn die Expansionskammer als zylindrische Ausnehmung ausgebildet ist und das Gaszuführungselement und das Gasabführungselement radial und einander gegenüberliegend in die Expansionskammer münden. Die zylindrische Gestaltung der Expansionskammer erlaubt die Realisierung eines vergleichsweise großen Volumens auf kleinem Raum. Es hat sich gezeigt, daß Expansionskammern mit einem Volumen, das etwa ein Drittel des Bauvolumens des Sensors beträgt, für eine ausreichende Dämpfung genügt.

Besonders günstig ist es, wenn der Gehäusedeckel einen Wandungsabschnitt in Form einer elastisch nachgebenden Ausgleichsmembran aufweist. Pulsationen oder Druckstöße im zugeführten Meßgasstrom werden durch eine korrespondierende elastische Verformung der Ausgleichsmembran zusätzlich kompensiert, so daß sich ein fast vollständiger Abbau der Druckstöße erreichen läßt. Auch läßt sich das Volumen der Expansionskammer reduzieren, da die Ausgleichsmembran einen Teil der Dämpfungsfunktion übernimmt.

Die Ausgleichsmembran ist bevorzugt einstückig mit dem Gehäusedeckel spritzgegossen, so daß sie mit vergleichsweise geringem Aufwand realisiert werden kann.

Weitere Varianten sind auf die Optimierung des Diffusionsmechanismus gerichtet, so daß ggfs. noch vorhandene, nicht vollständig abgebaute Druckstöße nicht auf die Sensorelektrode durchschlagen können.

Hierzu ist eine Diffusionsbariere in Form einer Scheibe vorgesehen, die am Gehäusedeckel im Bereich des Übergangs von der Expansionskammer zum Sensorelement angebracht ist. Die Scheibe besitzt axiale Durchgangsbohrungen geringen Durchmessers, die als Kapillaren wirken. Sie wirken damit als weiteres Dämpfungsglied für die in der Expansionskammer bereits abgeschwächten Druckstöße.

Eine zusätzliche Verbesserung ergibt sich dann, wenn eine Membran die Kapillarbohrungen zur Expansionskammer hin abdeckt. Die Membran ist demnach an der Oberseite der Diffusionsbariere befestigt und verhindert eine direkte Anströmung der Kapillarbohrungen.

Zusätzlich kann auch auf der gegenüberliegenden Seite an der Diffusionsbariere eine Diffusionsmembran angebracht sein, die damit die Schnittstelle zwischen dem eigentlichen Sensorelement und der Dämpfungsanordnung darstellt.

Aus dem vorstehenden ergibt sich, daß mit Hilfe des beschriebenen Konzepts ein äußerst kompakt aufgebauter Sensor mit integrierter Dämpfung für Druckstöße realisierbar ist, der eine problemlose Verwendung bei transportablen Gasanalyseeinrichtungen zuläßt. Speziell für diesen Anwendungsfall ist deshalb eine weitere bevorzugte Variante vorgesehen, bei der am Gehäuse eine elektronische Baugruppe integriert ist. Diese kann beispielsweise einen elektronischen Speicherbaustein (EEPROM) enthalten, der sämtliche, für den Betrieb des betreffenden Sensorelements relevanten Daten enthält. Mit dem Austausch des Sensors erfolgt zwangsläufig auch der Austausch des Speicherbausteins, so daß am Analysegerät keine weitere Aktualisierung vorgenommen werden muß. Damit sind Fehlmessungen oder Fehlbedienungen weitgehend ausgeschlossen, insbesondere wenn zur Herstellung der elektrischen Verbindung zum Analysegerät ein Steckverbinder vorgesehen ist.

Die Erfindung wird nachstehend näher anhand der in den Figuren dargestellten Ausführungsbeispiele erläutert. Es zeigen:
- Fig. 1: Meßanordnung,
- Fig. 2: Meßanordnung, Variante gemäß Fig. 1

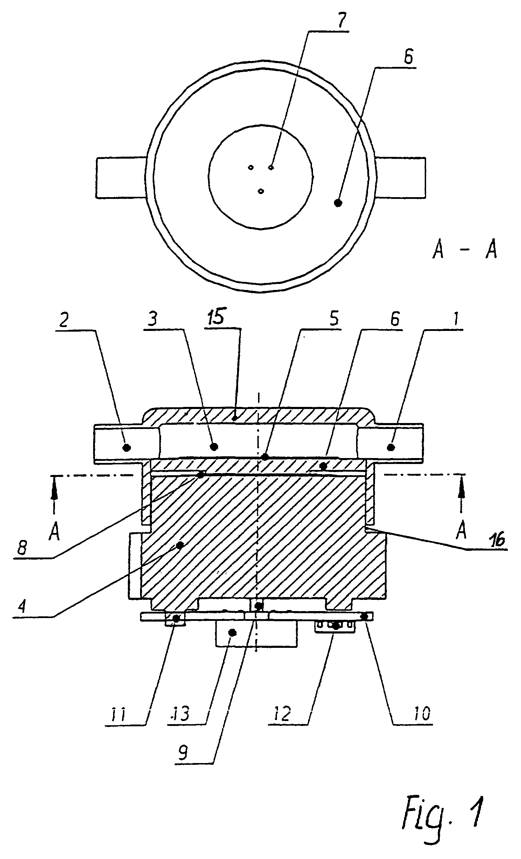

Der grundsätzliche Aufbau der erfindungsgemäßen Meßanordnung ergibt sich aus Fig. 1.

Ein an sich bekanntes und im Handel erhältliches Sensorelement 4 ist von einem Gehäuseunterteil 16 aufgenommen. Auf das Gehäuseteil 16 ist ein Gehäusedeckel 15 aufgesetzt, der ein Gaszuführungselement in Form eines Einlaßnippels 1 und ein Gasabführungselement in Form eines Auslaßnippels 2 aufweist. Der Einlaßnippel 1 und der Auslaßnippel 2 sind einander gegenüberliegend angeordnet und münden jeweils radial in eine zylindrische Ausnehmung 3, die innerhalb des Gehäusedeckels 15 eingeformt ist und als Expansionskammer für das durch den Einlaßnippel 1 zugeführte, druckstoßbehaftete Gas dient.

Nach unten, d.h. zum Sensorelement 4 hin gerichtet, ist in den Gehäusedeckel 15 eine Durchtrittsbarriere in Form einer Scheibe 6 eingesetzt. Die Durchtrittsbarriere weist drei axiale Durchgangsbohrungen 7 geringen Durchmessers auf, die als Kapillarbohrungen wirken. Zur Expansionskammer 3 hin ist die Durchtrittsbarriere 6 mit einer Membran 5 versehen, die zumindest den Bereich der Kapillarbohrungen 7 abdeckt und damit deren direkte Anströmung verhindert. Auf der gegenüberliegenden Seite, d.h. auf das Sensorelement 4 hin gerichtet, ist eine Diffusionsmembran 8 angebracht.

Im Gehäusedeckel 15 sind demnach praktisch sämtliche Bauteile enthalten, die die Dämpfung von Druckstößen bewirken. So sorgt die Expansionskammer 3 zunächst zu einer sehr starken Herabsetzung der Pulsationen des zugeleiteten Gasstroms, der zum größten Teil über den Auslaßnippel 2 wieder abgeführt wird. Lediglich eine vergleichsweise geringe Gasmenge gelangt über den eingangs beschriebenen Diffusionsmechanismus in das Innere des Sensorelements 4. Die Diffusionsbarriere 6 reduziert die noch verbliebenen Druck- und Dichteschwankungen, wozu die Membran 5 und die Diffusionsmembran 8 einen zusätzlichen Beitrag leisten. Der auf das Sensorelement 4 auftreffende (Teil-) Gasstrom ist soweit beruhigt, daß ggfs. noch vorhandene Druck- oder Dichteschwankungen keine unzulässige Abweichung im Meßsignal liefern.

Die Meßsignale werden in Form von sogenannten Rohsignalen über Anschlußstifte 9 bereitgestellt. Üblicherweise gelangen sie von dort über eine Leitung zur Auswerteeinheit, wo sie unter Berücksichtigung dort gespeicherter, sensorspezifischer Daten ausgewertet werden.

Im vorliegenden Ausführungsbeispiel werden die Rohsignale jedoch über die Anschlußstifte 9 auf eine Leiterplatte 10 übertragen, die mit Hilfe von an sich bekannten Formteilen 11 am Sensorelement 4 bzw. am Gehäuseunterteil 16 angebracht sind. Die Leiterplatte 10 ist mit elektrischen und elektronischen Bauteilen ausgestattet, die u.a. eine Identifikation des Sensortyps und eine Weiterverarbeitung des Sensorsignals ermöglichen. In diesem Zusammenhang ist ein integrierter elektronischer Speicherbaustein (EEPROM) 12 von Interesse, der die für den Betrieb des Sensors relevanten Daten, beispielsweise dessen Empfindlichkeit, enthält. Auf diese Weise entfällt nach einem Austausch des kompletten Sensors die sonst notwendige Aktualisierung von Sensordaten in der Auswerteeinheit. Dies ermöglicht Prüfung und Abgleich des Sensors getrennt und unabhängig vom Meßgerät, kann also beispielsweise werkseitig vorgenommen sein.

Die elektrische Kontaktierung des vorverarbeiteten Sensorsignals und des Speicherbausteins 12 erfolgt über einen gemeinsamen Steckverbinder 13.

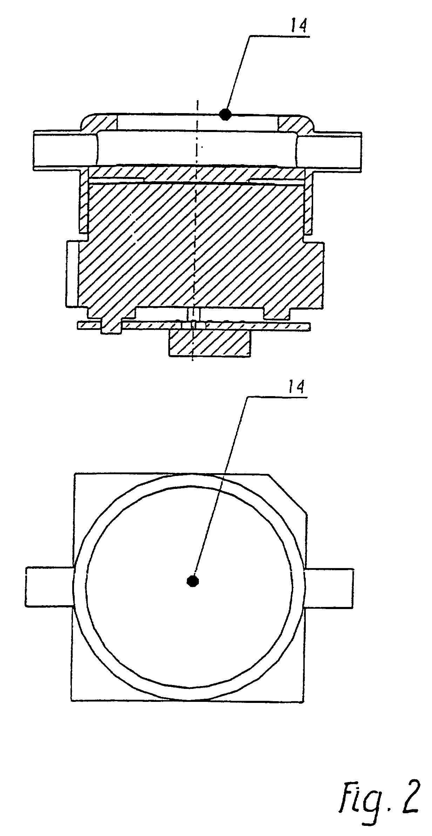

Die in Figur 2 dargestellte Variante unterscheidet sich von der vorhergehend beschriebenen dadurch, daß der Gehäusedeckel 15 eine Ausgleichsmembran 14 enthält. Diese erstreckt sich praktisch vollständig über die gesamte Stirnfläche des Gehäusedeckels 15 und ist integral im Spritzgießverfahren angeformt. Die Ausgleichsmembran 14 ist damit in der Lage, im Rhythmus der Druckstöße des zugeführten Gasstroms auszuweichen und gleicht damit Druckschwankungen innerhalb der Expansionskammer 3 weitgehend aus.

Diese Variante ist insbesondere dann vorteilhaft, wenn das Volumen der Expansionskammer 3 zu gering ist, um die Druckschwankungen der vorgeschalteten Pumpe ausreichend zu dämpfen. Sie erlaubt auch eine Reduzierung des Volumens der Expansionskammer 3, so daß die gesamte Meßanordnung insgesamt kleiner ausgelegt werden kann. Damit können auch solche Pumpen zum Einsatz kommen, die starke Druckstöße produzieren. Auch kurze Schlauchwege zwischen der Pumpe und dem Gassensor führen nicht zu unzulässigen Signalverfälschungen.

In einem konkreten Anwendungsfall ist es gelungen, durch konsequente Anwendung des erfindungsgemäßen Konzepts eine Schlauchlänge von 0,2 Metern (bei einem Innendurchmesser von 4 mm) ohne Einbuße an Meßgenauigkeit einzusparen. Dies bedeutet einen wesentlichen Fortschrit in Richtung auf ein gut handhabbares und leicht transportierbares Meßsystem, das einen sicheren Betrieb zuläßt.

### Bezugszeichenliste

- 1: Einlaßnippel
- 2: Auslaßnippel
- 3: Expansionskammer
- 4: Sensorelement
- 5: Membran
- 6: Durchtrittsbarriere
- 7: Kapillarbohrungen
- 8: Diffusionsmembran
- 9: Anschlußstift
- 10: Leiterplatte
- 11: Formteil
- 12: Speicherbaustein
- 13: Steckverbinder
- 14: Ausgleichsmembran
- 15: Gehäusedeckel
- 16: Gehäuseunterteil

## Patentansprüche

1. Messanordnung zur Untersuchung gasförmiger Medien mit
- einem Sensorelement (4), welches elektrische Signale entsprechend der Konzentration und/ oder der Zusammensetzung eines Gases erzeugt,
- einem Gaszuführungselement (1), über welches das Gas dem Sensorenelement zugeleitet wird,
- einem Gasabführungselement (2), über welches das Gas vom Sensorelement abgeleitet wird,
- einem Dämpfungselement (3), welches zuströmseitige Druckschwankungen des Gases dämpft, wobei das Dämpfungselement (3) Bestandteil eines das Sensorelement (4), das Gaszuführungselement (1) und das Gasabführungselement (2) aufnehmenden Gehäuses (15) und als Expansionskammer ausgebildet ist,
- **dadurch gekennzeichnet, dass** die Expansionskammer (3) in einem Gehäusedeckel (15) eingeformt ist, der auf ein das Sensorelement (4) aufnehmendes Gehäuseunterteil (16) aufgesetzt ist und das Gaszuführungselement (1) und das Gasabführungselement (2) trägt.

2. Messanordnung nach Anspruch 1, **dadurch gekennzeichnet, dass** die Expansionskammer (3) als zylindrische Ausnehmung ausgebildet ist, in die radial und einander gegenüberliegend das Gaszuführungselement (1) und das Gasabführungselement (2) münden.

3. Messanordnung nach Anspruch 1 oder 2, **dadurch gekennzeichnet, dass** der Gehäusedeckel (15) einen Wandungsabschnitt in Form einer elastisch nachgebenden Ausgleichsmembran (14) aufweist.

4. Messanordnung nach Anspruch 3, **dadurch gekennzeichnet, dass** die Ausgleichsmembran (14) einstückig mit dem Gehäusedeckel (15) spritzgegossen ist.

5. Messanordnung nach einem der Ansprüche 1 bis 4 , **gekennzeichnet durch** eine Diffusionsbarriere (6) in Form einer mit Kapillarbohrungen (7) versehenen Scheibe, die am Gehäusedeckel (15) am Übergang von der Expansionskammer (3) zum Sensorelement (4) angebracht ist.

6. Messanordnung nach Anspruch 5, **gekennzeichnet durch** eine Membran (5), die an der Diffusionsbarriere (6), der Expansionskammer (3) zugewandt, angebracht ist.

7. Messanordnung nach Anspruch 5 oder 6, **gekennzeichnet durch** eine Diffusionsmembran (8), die zwischen der Diffusionsbarriere (6) und dem Sensorelement (4) angeordnet ist.

8. Messanordnung nach einem der vorhergehenden Ansprüche, **dadurch gekennzeichnet, dass** am Gehäuse (16) eine elektronische Baugruppe (10) integriert ist, die vorzugsweise einen elektronischen Speicherbaustein (EEPROM) (12) mit für den Betrieb des Sensorelements (4) relevanten Daten enthält.

## Claims

1. A measurement arrangement for investigating gaseous media having
- a sensor element (4), which generates electrical signals corresponding to the concentration and/or the composition of a gas,
- a gas supply element (1), via which the gas is conveyed to the sensor element,
- a gas discharge element (2), via which the gas is carried away from the sensor element,
- a damping element (3), which dampens pressure fluctuations of the gas on the inflow side, the damping element (3) being constructed as a component of a housing (15) accommodating the sensor element (4), the gas supply element (1) and the gas discharge element (2) and as an expansion chamber,
- **characterised in that** the expansion chamber (3) is formed in a housing cover (15), which is placed onto a lower housing part (16) receiving the sensor element (4) and bears the gas supply element (1) and the gas discharge element (2).

2. A measurement arrangement according to Claim 1,
**characterised in that** the expansion chamber (3) is constructed as a cylindrical recess, into which the gas supply element (1) and the gas discharge element (2) open radially and mutually opposite.

3. A measurement arrangement according to Claim 1 or 2,
**characterised in that** the housing cover (15) comprises a wall portion in the form of an elastically yielding compensating membrane (14).

4. A measurement arrangement according to Claim 3,
**characterised in that** the compensating membrane (14) is injection moulded in one piece with the housing cover (15).

5. A measurement arrangement according to one of Claims 1 to 4,
**characterised by** a diffusion barrier (6) in the form of a disk provided with capillary bores (7), which is mounted on the housing cover (15) at the transition from the expansion chamber (3) to the sensor element (4).

6. A measurement arrangement according to Claim 5,
**characterised by** a membrane (5), which is mounted on the diffusion barrier (6), facing the expansion chamber (3).

7. A measurement arrangement according to Claim 5 or 6,
**characterised by** a diffusion membrane (8), which is disposed between the diffusion barrier (6) and the sensor element (4).

8. A measurement arrangement according to one of the preceding Claims,
**characterised in that** an electronic module (10), which preferably contains an electronic memory module (EEPROM) (12) with data relevant for the operation of the sensor element (4), is integrated on the housing (16).

## Revendications

1. °)Dispositif de mesure pour examiner des milieux gazeux, comportant :
- un élément capteur (4) qui crée des signaux électriques en fonction de la concentration et/ou de la composition d'un gaz,
- un élément d'amenée des gaz (1) conduisant le gaz à l'élément capteur,
- un élément d'évacuation des gaz (2) évacuent le gaz de l'élément capteur,
- un élément amortisseur (3) qui amortit les fluctuations de pression du côté de l'amenée des gaz, l'élément amortisseur (3) étant formé comme chambre de détente faisant partie d'un boîtier (15) logeant l'élément capteur (4), l'élément d'amenée des gaz (1) et l'élément d'évacuation des gaz (2),
**caractérisé en ce que**
la chambre de détente (3) est moulée dans un couvercle de boîtier (15) qui est posé sur une pièce inférieure du boîtier (16) logeant l'élément capteur (4), et qui porte l'élément d'amenée des gaz (1) et celui d'évacuation des gaz (2).

2. °)Dispositif de mesure selon la revendication 1,
**caractérisé en ce que**
la chambre de détente (3) est formée comme un évidement cylindrique dans lequel débouchent l'élément d'amenée des gaz (1) et celui d'évacuation des gaz (2) radialement et en face l'un de l'autre.

3. Dispositif de mesure selon la revendication 1 ou 2,
**caractérisé en ce que**
le couvercle de boîtier (15) présente une section de paroi en forme de membrane de compensation (14) élastique extensible.

4. Dispositif de mesure selon la revendication 3,
**caractérisé en ce que**
la membrane de compensation (14) est moulée par injection d'un seul tenant avec le boîtier de couvercle (15).

5. Dispositif de mesure selon l'une quelconque des revendications 1 à 4,
**caractérisé par**
une barrière de diffusion (6) en forme de disque muni d'alésages capillaires (7), appliquée sur le passage allant de la chambre de détente (3) à l'élément capteur (4) sur le couvercle de boîtier (15).

6. Dispositif de mesure selon la revendication 5,
**caractérisé par**
une membrane (5) appliquée sur la barrière de diffusion (6) opposée à la chambre de détente (3).

7. Dispositif de mesure selon la revendication 5 ou 6,
**caractérisé par**
une membrane de diffusion (8) disposée entre la barrière de diffusion (6) et l'élément capteur (4).

8. Dispositif de mesure selon l'une quelconque des revendications précédentes,
**caractérisé en ce qu'**
un bloc (10) électrique est intégré sur le boîtier (16), lequel bloc contient de préférence un module de mémoire électronique (EEPROM) (12) avec les données opportunes pour l'exploitation de l'élément capteur (4).
